# EUROPEAN PATENT SPECIFICATION

(11) **EP 1 465 530 B1**
(45) Date of publication and mention of the grant of the patent: **23.05.2007**
(21) Application number: 03704951.7
(22) Date of filing: 05.01.2003
(51) Int. Cl.: A61B 8/00, A61B 8/08, G01N 29/24

(54) **A SYSTEM AND METHOD OF MAPPING IRREGULARITIES OF HARD TISSUE**
SYSTEM UND VERFAHREN ZUR KARTIERUNG VON UNREGELMÄSSIGKEITEN VON HARTEM GEWEBE
SYSTEME ET PROCEDE DE CARTOGRAPHIE D'IRREGULARITES DE TISSU DUR

(43) Date of publication of application: 13.10.2004
(73) Proprietor: GE Medical Systems Israel Ltd., 39120 Tirat HaCarmel (IL)
(72) Inventor: SELA, Natan, 76702 Rehovot (IL); BUKSHPAN, Shmuel, 76702 Rehovot (IL); COHEN, Lior, 76702 Rehovot (IL); KARDOSH, Michael, Rehovot 76702 (IL)
(74) Representative: Pedder, James Cuthbert
(86) International application number: PCT/IL2003/000016
(87) International publication number: WO 2003/057001

(56) References cited:
- WO-A-97/13145
- US-A- 5 197 475
- US-A- 5 235 981
- US-A- 5 293 870
- US-A- 5 749 364

## Description

This application claims priority from United States Patent application 60/344,803 filed on January 07, 2002 and from United States Patent application 60/361,091 filed on March 01, 2002

### FIELD AND BACKGROUND OF THE INVENTION

The present invention relates to a system and method of mapping irregularities of hard tissue. Specifically, the invention relates to detecting irregularities in hard tissue such as bone (e.g. fractures, joint abnormalities and implanted surgical anchors.)

According to the American Academy of Orthopaedic Surgeons, every year approximately 6.5 million bone fracture cases are diagnosed in the United States alone. Orthopedic medicine has traditionally relied upon radiographic images (e.g. X-Ray or CT scan) of bone tissue as a means of diagnosing bone abnormalities including fractures and malformations. These methods require exposing a patient to radiation.

U.S. Patent 4,798,210 issued to Ledley describes a method for developing a 3D image of a 3D object using ultrasound whereby a first image is combined with a second image in order to create a 3D image. Again, teachings of Ledley contain neither a hint nor suggestion that renderings of bone or imperfections therein may be produced by ultrasound.

U.S. Patent 5,924,989 issued to Polz is an additional example of a three dimensional ultrasonic system for capturing images of dynamic organs such as the heart or other parts of the respiratory system. Like Ledley, Polz employs a combination of different images in order to complete the three dimensional image. Again, teachings of Polz contain neither a hint nor suggestion that renderings of bone or imperfections therein may be produced by ultrasound.

U.S. Patent 5,928,151 issued to Hossack et al. is a further example of a three dimensional ultrasonic scanning system. Again, teachings of this patent contain neither a hint nor suggestion that renderings of bone or imperfections therein may be produced by ultrasound. The opposite is true, the emphasis on the ability to work without contrast agents suggests that Hossack envisioned only soft tissue applications.

U.S. Patent 6,120,453 issued to Sharp is a three-dimensional ultrasound system. The teachings of this patent are similar to those of Ledley and Polz. Again, Sharp employs the combination of several images to create a three dimensional image. Again, Sharp offers neither a hint nor suggestion that renderings of bone or imperfections therein may be produced by ultrasound.

In summary, none of the patents in this first group even imply that generation maps of irregularities of bone can be generated using ultrasound technology. Instead, they stress various means of increasing resolution of 3D images of soft tissue. Application of these methods directly to hard tissue is impractical because the echo reflection properties of soft tissue are not similar to those of hard tissue.

The concept of ultrasonic imaging of bone is also not unknown. However, bone images produced by ultrasound are typically not three dimensional as exemplified by this second group of prior art references.

U.S. Patent 4,476,873 issued to Sorenson et al. is an ultrasound scanning system used for imaging skeletal structure. This scanning system can distinguish between hard and soft tissue and is used to detect scoliosis. However, figures 14-18 of this patent make it abundantly clear that while data may be collected in three dimensions, output is supplied as graphs. Thus, it is an inherent disadvantage of Sorenson that images are not provided as a result of the scan. Sorenson teaches differentiation between lungs containing air and bones. It will be appreciated that lung tissue, which presents alternating layers of air and soft tissue, is more different from bone than other soft tissues such as muscle. Further, Sorenson teaches that Snell's law typically causes most transmitted energy to be reflected along a line which is at an angle to a longitudinal axis of the transmitting transducer. Therefore, Sorenson teaches extensive amplification of the small amount of reflected energy returning along this axis or, in the alternative, capture of reflected energy at one or more additional transducers. Thus, Sorenson teaches determination of co-ordinates of a point in three degrees of freedom, as opposed to six degrees of freedom. Thus changes in an angle of a surface over distance are not determined by these teachings. This is a distinct and inherent disadvantage which renders these teachings unsuitable to use in imaging surface irregularities of long bones.

U.S. Patent 5,140,988 issued to Stouffer et al. is a method and apparatus for imaging bone structures in animal carcasses. Figures 2 and 3 of this patent demonstrate that the teachings of Stouffer relate to 2 dimensional images of bone. Stouffer fails to teach imaging of irregularities in bone surface such as fractures.
U.S. Patent 5,840,029 issued to Mazess et al is a method for using ultrasound to measure bone. Mazess concerns himself primarily with measurement of bone properties. Mazess fails to teach imaging of irregularities in bone surface such as fractures.

U.S. Patent 5,879,301 issued to Chibrera et al. is a method for detecting the properties of bone using ultrasound, specifically for detecting osteoporosis. It is an inherent disadvantage of Chibrera that production of images of measured bones, or surface irregularities thereof is not taught.

U.S. Patent 6,015,383 issued to Buhler et al. teaches acoustic analysis to detect the characteristics of bone tissue where the edge of the bone is detected. However, figures 3-6 of this patent make it abundantly clear that output is supplied as graphs. Thus, it is an inherent disadvantage of Buhler that images are not provided as a result of the scan.

U.S. Patent 322,507 issued to Passi et al. is an ultrasonic system for evaluation of bone tissue. Like other patents in this group, it has the inherent disadvantage of providing output as graphs rather than images. Further, measurements according to these teachings are of acoustic properties and not of surface position co-ordinates.

Thus, while members of this second group of patents teach assays of bone using ultrasound technology, they fail to teach production of maps of surface irregularities in bone.

Additional patents dealing with ultrasonic imaging of bone are presented hereinbelow.

U.S. Patent 5,305,752 issued to Spivey is a system for imaging tissue in the body using acoustic waves. While Spivey teaches formation of a single ultrasonic image depicting both soft tissue and bone, the image is a cross-sectional image (i.e. 2 dimensional). Spivey does not teach imaging of surface irregularities such as fractures.

U.S. Patent 5,465,722 issued to Fort et al. relates to a 3D ultrasonic system. Although these teachings included production of a 3D image of a bone (Figure 13), they do not include imaging of surface irregularities such as fractures. U.S. Patent 6,375,616 issued to Soferman et al. is a method for determining fetal weight in utero. Although Soferman teaches application of grey level threshold in order to isolate bones from other tissue in an image, his teachings do not include imaging of surface irregularities such as fractures.

U.S. Patent 6,390,982 issued to Bova et al. is a method of creating a three dimensional image. The teachings of Bova are directed to ultrasonic probes as an adjunct to a second imaging technology in localizing bone. This means that generation of the three dimensional image from ultrasound image data alone is beyond the scope of Bova's teachings. Further, Bova does not teach imaging of surface irregularities such as fractures.

U.S. Patent 6,413,215 issued to Wu et al. is an ultrasonic system for detecting the wear of artificial joints. The teachings of Wu rely upon scattering of ultrasonic energy as a result of cavitation events in synovial fluid. Further, Wu teaches output of data as particle size information, not particle position. In summary, these teachings have little relevance to the instant application because cavitation events are not expected to occur in typical measurement of hard tissue surface irregularities.

US 5,235,981 relates to a method and apparatus for detecting and locating a bony region by ultrasound. US 5,235,981 mentions determining a transient disturbance in an echographic signal as a way of determining a bony discontinuity.

US 5,197,475 discloses apparatus for investigating the mechanical properties of a solid material such as bone. The apparatus permits evaluation of treatment for osteoporosis, and whether that treatment has in fact reduced the tendency of a patient's bones to fracture.

WO 97/13145 discloses a method for determining, through an interposing medium, the mechanical properties of a solid having a surface.

### SUMMARY OF THE INVENTION

According to one aspect of the present invention there is provided a method of mapping irregularities in a surface of a hard tissue within a target. The method includes: (a) transmitting from an ultrasonic transducer at a defined location a focused beam of ultrasonic energy towards the surface of the hard tissue at a first oblique angle of incidence; (b) registering the defined location if and only if an echo-reflection from the surface of the hard tissue is received by the transducer; (c) defining the location of the transducer in six degrees of freedom; (d) calculating a set of position co-ordinates for a portion of the surface of the hard tissue causing the echo-reflection; (e) moving the ultrasonic transducer to a different defined location; (f) repeating (a) through (e); (g) repeating steps (a) through (f) with an additional angle at least 20 degrees apart from the previous oblique angle; (h) determining for each of said set of position co-ordinates for a portion of the surface of the hard tissue causing said echo-reflection at least a portion of a reflection diagram (i) further determining for each of said at least a portion of a reflection diagram a degree of normalcy according to a predetermined rule to generate a map of the irregularities in the surface of the tissue; (j) classifying any of said set of position co-ordinates for a portion of the surface of the hard tissue wherein said at least a portion of a reflection diagram is characterized by a low degree of normalcy according to said predetermined rule as belonging to a surface irregularity; and (k) compiling at least a portion of the sets of position co-ordinates to generate a map of the irregularities in the surface of the hard tissue.

According to another aspect of the present invention there is provided a system for mapping irregularities in a surface of a hard tissue within a target. The system includes at least one ultrasonic transducer, a position locator and adjustment mechanism and a central processing unit. The at least one transducer is positioned at a defined location and is capable of transmitting a focused beam of ultrasonic energy towards the surface of the hard tissue at a first oblique angle of incidence and is further capable of receiving at least a portion of the energy as an echo-reflection from the surface of the hard tissue and is further capable of communication with the central processing unit. The position locator and adjustment mechanism is operably connectable to the at least one transducer and is designed and constructed to be capable of adjusting the oblique angle of incidence between the focused beam and the surface of the hard tissue in response to a command from the central processing unit; and to be further capable of defining the location of the transducer as a set of position co-ordinates in six degrees of freedom and transmitting the set of co-ordinates to a central processing unit and to be further capable of moving the at least one ultrasonic transducer to a series of different defined location. The central processing unit designed and configured to be capable of receiving the set of position co-ordinates defining the location of the at least one transducer from the position locator and adjustment mechanism and to be further capable of calculating an additional set of position co-ordinates for a portion of the surface of the hard tissue causing the echo-reflection and to be further capable of compiling a plurality of the sets of position co-ordinates to generate a map of the surface of the hard tissue.

According to further features in preferred embodiments of the invention described below, the method further includes: (h) determining for each of the set of position co-ordinates for a portion of the surface of the hard tissue causing the echo-reflection at least a portion of a reflection diagram; (i) further determining for each of the at least a portion of a reflection diagram a degree of normalcy according to a predetermined rule to generate a map of the irregularities in the surface of the tissue; and (j) classifying any of the set of position co-ordinates for a portion of the surface of the hard tissue wherein the at least a portion of a reflection diagram is characterized by a low degree of normalcy according to the predetermined rule as belonging to a surface irregularity.

According to still further features in the described preferred embodiments repeating includes adjusting the oblique angle of incidence to at least one second oblique angle of incidence.

According to still further features in the described preferred embodiments the method further includes controlling, by means of a central processing unit, performance of at least a portion of the method.

According to still further features in the described preferred embodiments controlling includes at least one item selected from the group consisting of the adjusting and the registering.

According to still further features in the described preferred embodiments controlling indicates at least one control mechanism selected from the group consisting of mechanical control, selection from an array and electronic control.

According to still further features in the described preferred embodiments at least one item selected from the group consisting of the adjusting and the registering is performed manually by a practitioner of the method.

According to still further features in the described preferred embodiments the central processing unit is further designed and constructed to be capable of transmitting a command to the position locator and adjustment mechanism to cause the at least one transducer to move to the series of different defined locations;

According to still further features in the described preferred embodiments the command is selected from the group consisting of a command to a mechanical control, a command to switch to a different transducer of the at least one transducer and a command to an electronic control.

According to still further features in the described preferred embodiments the position locator and adjustment mechanism is designed and configured to receive input from an operator of the system, the input being selected from the group consisting of a manual position adjustment by an operator of the system and at least one instruction transmitted to the central processing unit by the operator.

According to still further features in the described preferred embodiments method further includes displaying upon a display device at least one item selected from the group consisting of: (i) data pertaining to the echo-reflection; (ii) the set of position co-ordinates for the portion of the surface of the hard tissue causing the echo-reflection; and (iii) at least a portion of the map.

According to still further features in the described preferred embodiments the map is a two dimensional map.

The present invention successfully addresses the shortcomings of the presently known configurations by providing a system and method of mapping irregularities of hard tissue such as imperfections in bone (e.g. fractures, joint abnormalities and implanted surgical anchors.)

Implementation of the method and system of the present invention involves performing or completing selected tasks or steps manually, automatically, or a combination thereof. Moreover, according to actual instrumentation and equipment of preferred embodiments of the method and system of the present invention, several selected steps could be implemented by hardware or by software on any operating system of any firmware or a combination thereof. For example, as hardware, selected steps of the invention could be implemented as a chip or a circuit. As software, selected steps of the invention could be implemented as a plurality of software instructions being executed by a computer using any suitable operating system. In any case, selected steps of the method and system of the invention could be described as being performed by a data processor, such as a computing platform for executing a plurality of instructions.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
FIG. 1 is a simplified flow diagram showing a sequence of events performed in execution of a method according to the present invention.
FIG. 2 is a schematic representation of a system according to the present invention.
FIGs. 3a-c illustrate arrangement of ultrasonic transducers in arrays for use in the context of the present invention.
FIGs. 4a - 4d illustrate echo reflection of an ultrasound beam transmitted through soft tissue toward a hard tissue according to the present invention.
FIGs. 5a - 5d illustrate reflection diagrams resulting from reflections presented in figure 4.

### DESCRIPTION OF THE PREFERRED EMBODIMENTS

The present invention is of a system and method of mapping irregularities of hard tissue that can be used to detect imperfections in bone.

Specifically, the present invention can be used to provide maps of fractures, joint abnormalities and implanted surgical anchors

The principles and operation of a system and method according to the present invention may be better understood with reference to the drawings and accompanying descriptions.

Before explaining at least one embodiment of the invention in detail, it is to be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

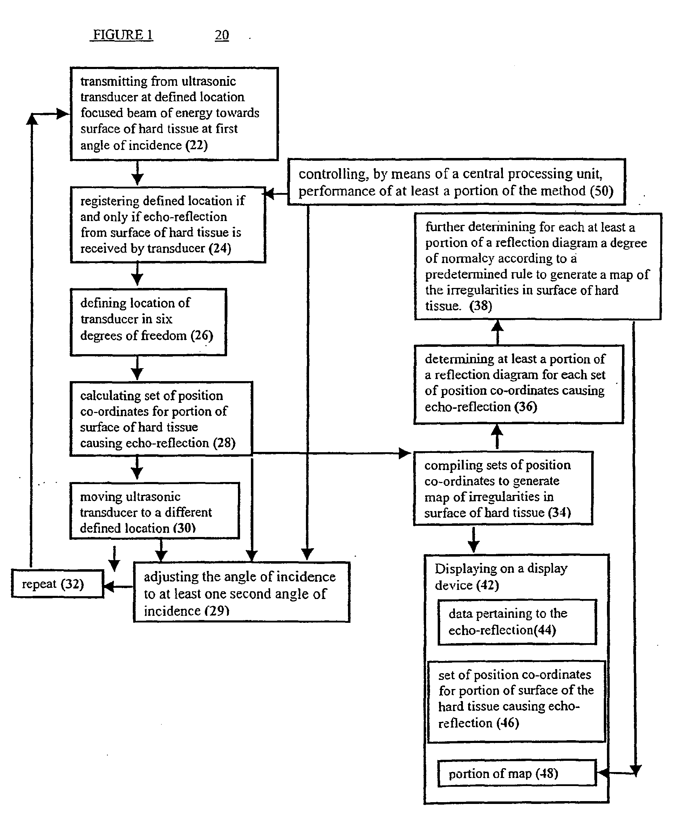

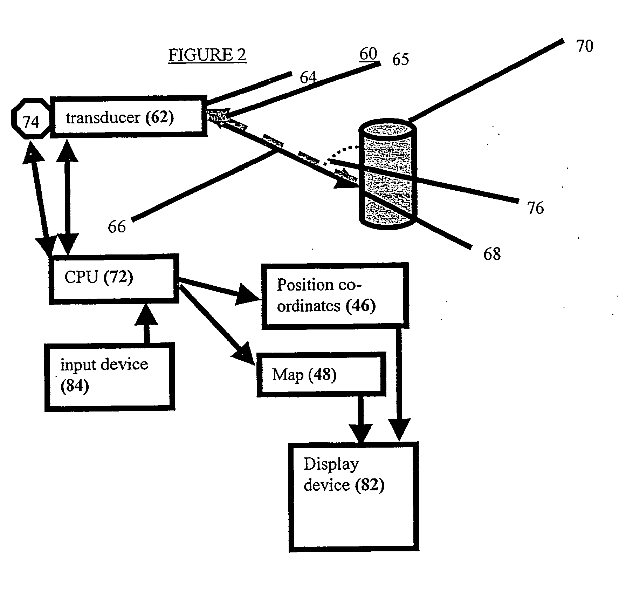

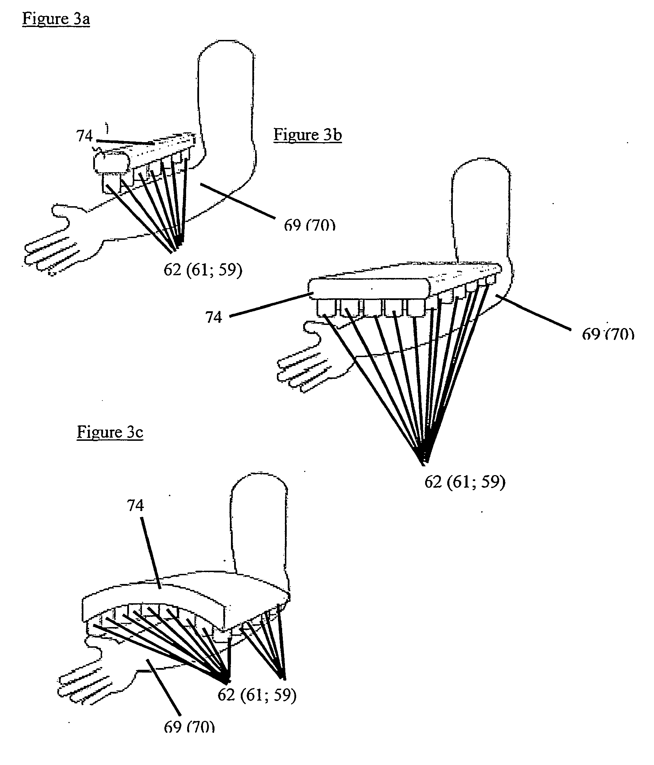

The present invention is preferentially embodied by a method **20** (figure 1) of mapping irregularities in a surface **68** (figure 2) of a hard tissue **70** within a target **69** (figure 3). Target **69** may include, for example, skin, fat, muscles, blood vessels, internal organs, and any other substantially soft, fluid, or low density biological tissue. Method **20** includes transmitting **22** from an ultrasonic transducer **62** at a defined location **64** a focused beam **66** of ultrasonic energy towards surface **68** of hard tissue **70** at a first oblique angle **76** of incidence.

For purposes of this specification and the accompanying claims, the term "ultrasonic" pertains to sound waves with a frequency in excess of approximately 20 kHz, more preferably in the range of 1 MHz to 20 MHz.

For purposes of this specification and the accompanying claims, the term "focused" means that a majority of transmitted energy is concentrated within a defined area surrounding an axis of a transmitted beam. Focusing may be caused by interference of a plurality of transmitted beams. Depending upon the arrangement of the transmitting transducers, this interference may involve a temporal component (i.e. transducers located further from the target transmitting earlier, those located closer to the target transmitting later) as well as a spatial component.

For purposes of this specification and the accompanying claims, the term "beam" indicates a ray of energy transmitted from one or more sources.

According to preferred embodiments of the invention, beam **66** is preferably a pulsed beam. For purposes of this specification and the accompanying claims, the term "pulsed" means temporally defined.

For purposes of this specification and the accompanying claims, the term "hard tissue" includes, but is not limited to bone such as cortical bone or trebicular bone. Bone refers to calcified fully developed bone capable of generating an ultrasonic echo-reflection in approximate accordance with Snell's law. While a developing fetus may have bones, these fetal bones are excluded from the definition of hard tissue because they are primarily cartilaginous, significant calcification typically taking place well after parturition.

Method **20** further includes registering **24** defined location **64** if and only if an echo-reflection **65** from surface **68** of hard tissue **70** is received by transducer **62.**

Method **20** further includes defining **26** location **64** of transducer **62** in six degrees of freedom.

Method **20** further includes calculating **28** a set of position co-ordinates **46** for a portion of surface **68** of hard tissue **70** causing echo-reflection65.

Method **20** further includes moving **30** ultrasonic transducer **62** to a different defined location **64.**

Method **20** further includes repeating **32** the sequence of transmitting **22,** registering **24,** defining **26** and calculating **28.** Repeating **32** may include, but is not limited to, adjusting **29** oblique angle of incidence **76** to at least one second oblique angle of incidence **76.** Additional angle **76** is preferably at least 20 degrees apart from first angle **76.**

Method **20** further includes compiling at least a portion of the sets of position co-ordinates **46** to generate a map **48** of the irregularities in surface **68** of hard tissue **70.**

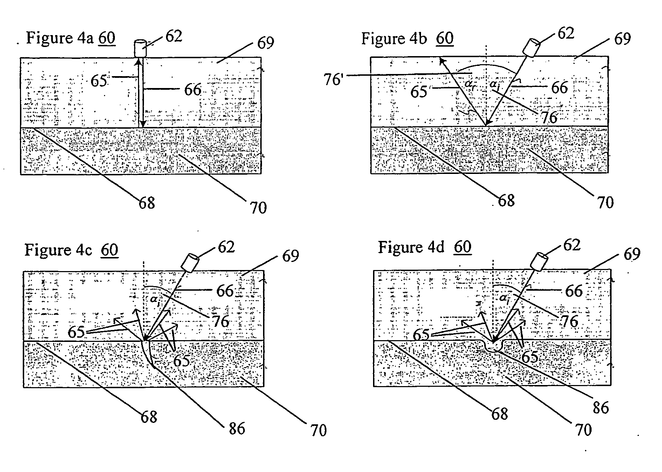

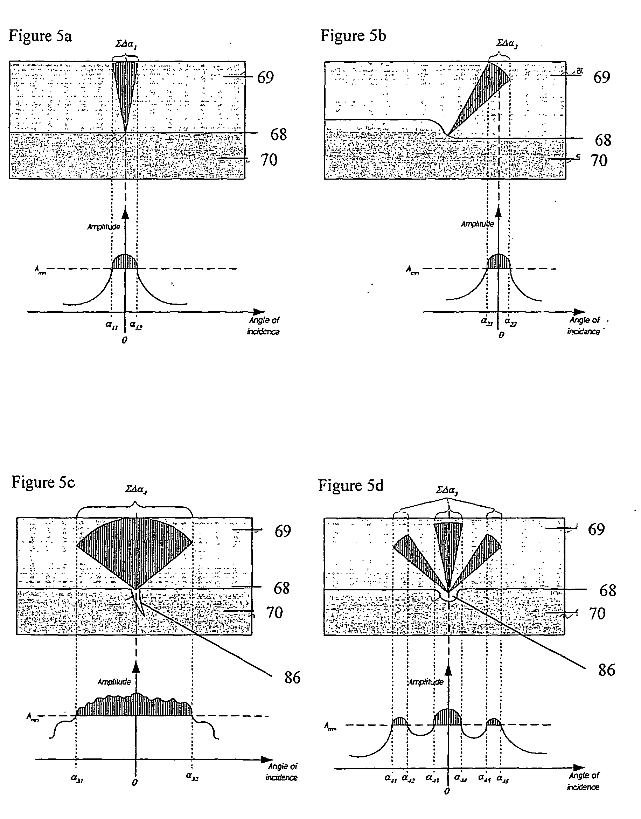

Preferably, method **20** further includes determining **36** for each set of position co-ordinates **46** for a portion of surface **68** of hard tissue **70** causing echo-reflection **65** at least a portion of a reflection diagram. For purposes of this specification and the accompanying claims, the phrase "reflection diagram" of a point on surface **68** of hard tissue **70** refers to a diagram illustrating the ratio between reflected energy **65** and transmitted energy **66** of an ultrasound beam transmitted towards that point on surface **68** of hard tissue **70** as a function of the different angles of incidence **76** between beam **66** and surface **68.** Examples of ways in which reflection diagrams of this type may be useful in mapping irregularities in surface 68 are presented in figures 4a-d and 5 a-d.

Method **20** and system **60** rely upon the fact that surface **68** of hard tissue **70** may be considered a specular reflector (figure 4a) provided that it does not include any corrugations **(86;** figures 4c, 4d, 5b and 5d) on a scale comparable with the wavelength of ultrasonic beam **66** or lower. If surface **68** of hard tissue **70** exhibits one or more irregularities **86,** such as pathological discontinuities or inhomogeneities such as a fracture, rupture, crack, or tear (figure 4c and 5c). Irregularity **86** may cause ultrasound beam **66** to be redirected or scattered to various directions **(65;** figures 4c and 4d and dark shaded areas; figures 5a-5d) possibly including toward ultrasound source **62,** in addition to, or instead of, specular reflection.

Hard tissue **70** includes any biological material which is, under normal conditions, substantially in a solid state. In humans, hard tissue **70** includes, for example, bones, cartilage, tendons, teeth, and nails. In nonhuman organisms hard tissue **70** further includes, but is not limited to, tusks, horns, claws and shells.

Irregularities **86** in surface **68** of hard tissue **70** may be caused by a wide variety of pathologies. These pathologies include, but are not limited to, pathological discontinuities and inhomogeneities, for example, fractures, ruptures, cracks and tears. Human bone fractures, for instance, frequently occur due to a trauma event (e.g. fall, blow or other impact), due to ongoing stress exerted on the bone (e.g. athletic or military activity), or due to bone deterioration. Bone deterioration (e.g. osteoporosis) is often a result of aging..

FIGS. 4a - 4d illustrate various examples of the path of an ultrasound beam **66** transmitted through a soft tissue target **69** toward a hard tissue **70** under examination. For illustration purposes, beam **66** is portrayed as a line (arrow indicates direction of wave propagation), although it is understood that in reality beam **66** has a certain width or volume which may furthermore change as beam **66** progresses.

Referring now to FIG. 4a, it is well known that if ultrasound beam **66** is transmitted perpendicularly, i.e. in a normal incidence, to surface **68** of hard tissue **70** whose dimensions are greater than a width of beam **66,** then the ultrasonic energy will be partially reflected **65** toward ultrasound source **62.**

Referring now to FIG. 4b, it is also well known that, in general, as the angle of incidence **76** αᵢ (relative to the normal incidence) is increased, the amount of energy reflected **65** toward ultrasound source **62** decreases. If the angle of incidence **76** αᵢ is substantially different than 0° (the normal incidence), then energy will be reflected **65** away from source **62** in an angle of reflection **76'** α*ᵣ* which is substantially equal to angle of incidence 76 α*ᵢ*. The reflection mentioned in connection to FIGS. 4a and 4b is often referred to as "specular reflection". A surface **68** of hard tissue **70** may be considered a specular reflector provided that it does not include any corrugations on a scale comparable with the wavelength of ultrasonic beam **66** or lower.

Referring now to figures 4c and 4d, if the hard tissue **70** exhibits one or more surface irregularities **86** (e.g. pathological discontinuity, inhomogeneity, fracture, rupture, crack, or tear), irregularity **86** may cause reflection **65** of beam **66** to be redirected or scattered to various directions possibly including toward ultrasound source **62**. This scattering is referred to as "nonspecular reflection".

In reducing the present invention to practice, it has been determined that the degree to which reflection **65** is scattered (i.e. nonspecular) is indicative of the nature of irregularity **86**. Conversely, the degree to which reflection **65** is specular is indicative of the smoothness of surface **68**.

Referring now to figure 4d, it should be noted that surface **68** of even a healthy hard tissue **70** is not perfectly flat, but rather exhibits local convexities, concavities, curves, and other types of geometries in various sizes and shapes which are attributable to the natural anatomy of hard tissue **70**. Such anatomical geometries, though not related to any pathology, may also cause reflection **65** of beam **66** to be redirected or scattered to various directions possibly including toward ultrasound source **62**.

Reflections **65** of ultrasonic energy **66** from a pathological discontinuity or inhomogeneity **86** (figure 4c), whether specular or nonspecular will be referred to hereinunder as "pathology echoes", and ultrasonic energy reflected, specularly or nonspecularly, from an anatomical geometry **86** (figure 4d), will be referred to hereinunder as "geometrical echoes".

Since echo reflections **65** from a healthy and substantially flat surface **68** of hard tissue **70** are primarily specular (i.e. their reflection is primarily limited to a certain angle of reflection) whereas pathology echoes and geometrical echoes are often both specular and nonspecular, it follows that reflection diagrams (as defined hereinabove) of a pathological discontinuity (**86**; figures 4c and 5c) and an anatomical geometry (**86**; figures 4d and 5d) are each differentiable from a reflection diagram of a healthy and substantially flat hard tissue **70**.

The extent of nonspecular reflection caused by a certain reflector on surface 68 of hard tissue 70, such as a pathological discontinuity or an anatomical geometry, depends on several factors. Those factors include, but are not limited to the size, shape and smoothness or roughness of the reflector, the wavelength of incident beam 66, and angle of incidence 76. Pathological discontinuities and inhomogeneities such as fractures have a typically different size, shape and roughness than anatomical geometries. Thus, in reducing the present invention to practice, it has been determined that reflection diagrams of pathological discontinuities and inhomogeneities are differentiable from those of anatomical geometries.

Figure 5 a to 5d illustrate examples of reflection diagrams of various reflectors on surface 68 of hard tissue 70. In the drawings, each dark area represents a range of incidence angles where specular or nonspecular reflection toward the ultrasound source approaches a maximum. It is understood that although, for illustration purposes, the diagrams are portrayed in two dimensions only, in reality reflection occurs in three dimensions; thus, for example, what appears in the drawings as a segment of a circle may resemble in reality a segment of a hemisphere.

Referring now to figure 5a, there is illustrated an example of a reflection diagram of a healthy and substantially flat area on surface 68 of hard tissue 70. In such a case, the maximal amplitude of returning echo 65 (figure 4a) is around the normal incidence that is, in the current example, between angles of incidence α*₁₁* and α*₁₂*. Therefore, it is evident that reflection from a substantially flat reflector is strongly angle-dependent.

Referring to figure 5b, there is illustrated an example of a reflection diagram of a slightly curved area on surface 68 of hard tissue 70. As long as the gradient about the area of incidence is low relative to the wavelength of incident beam 66 and provided that the local surface is sufficiently smooth, the maximal amplitude of the returning echo remains around the locally defined normal incidence that is, in the current example, between *α₂₁* and *α₂₂.* Thus, it can be seen that reflection from a curved surface that is characterized with a low gradient relative to the wavelength of the ultrasound beam, is strongly angle-dependent like the reflection diagram produced by a flat surface.

Referring to figure 5c, there is illustrated an example of a reflection diagram of a pathological discontinuity 86 on surface 68 of hard tissue 70. Discontinuity 86 may include, for example, a fracture, rupture, crack, tear or other pathology recited hereinabove. The present invention relies on the principle that the reflection diagram of pathological discontinuity **86** remains approximately maximum throughout a relatively wide range of angles of incidence such as, in the current example, from α₃₁ through α₃₂. Moreover, scattering caused by pathological discontinuity **86** tends towards uniformity throughout a relatively wide range of angles of incidence (α). Thus, pathological discontinuity is characterized by a reflection diagram which is largely angle independent.

Referring to figure 5d, there is illustrated an example of a reflection diagram of an anatomical geometry **86** on surface **68** of hard tissue **70**, for example, a local concavity. The reflection diagram of anatomical geometries in healthy hard tissue **70** typically exhibit one or more distinct local extrema such as, in the current example, a first local maximum between α*₄₁* and *α₄₂*, a second local maximum between α*₄₃* and *α₄₄*, and a third local maximum between α*₄₅* and *α₄₆*. Thus, nonspecular reflection from a healthy hard tissue **70** surface which includes an anatomical geometry **86** is angle-dependent like the flat or slightly curved surfaces of figures 5a and 5b, although to a lesser degree.

In summary, the reflection diagram of a pathological discontinuity or inhomogeneity **86** on surface **68** of hard tissue **70** is typically wider and more uniform, i.e. less angle-dependent, than the reflection diagram of a healthy hard tissue **70**. This is true even if the reflection diagram is compared to one resulting from an anatomical geometry. More specifically, a pathological discontinuity or inhomogeneity causes nonspecular reflection toward the ultrasound source over a wider range of angles of incidence than does healthy tissue with or without an anatomical geometry. Further, nonspecular reflection from a pathological discontinuity or inhomogeneity remains near maximum (i.e. is largely angle independent) throughout a relatively wide range of angles of incidence, whereas reflection from an anatomical geometry typically exhibits one or more local extrema (i.e. is largely angle dependent).

It is therefore a particular and fundamental feature of the present invention that it enables detection, and consequently location, mapping and imaging of pathological discontinuities and inhomogeneities on a surface **68** of hard tissue **70** based upon analysis of reflection diagrams thereof and comparison to reflection diagrams produced by adjacent surface 68 of healthy hard tissue **70**.

Preferably, method **20** includes further determining **38** for each of the at least a portion of a reflection diagram a degree of normalcy according to a predetermined rule to generate a map of the irregularities in surface **68** of hard tissue **70**. Thus, method **20** optionally but preferably includes classifying any of position co-ordinates **46** for surface **68** of hard tissue **70** wherein the at least a portion of a reflection diagram is characterized by a low degree of normalcy according to the predetermined rule as belonging to a surface irregularity.

Preferably, method **20** further includes controlling **50,** by means of a central processing unit **72,** performance of at least a portion of method **20.** Controlling **50** include, for example, adjusting **29** or registering **24.** Controlling **50** may be accomplished, for example, employing a mechanical control mechanism, selection from an array or electronic control. Examples of arrays of transducers **62** suited for use in the present invention are presented in figures 3a-c. Use of arrays to accomplish moving **30** greatly increases the speed with which position co-ordinates **46** and map **48** may be generated. Optionally, but also preferably, portions of method **20** (e.g. adjusting **29** and/or registering **24)** may be performed manually by a practitioner thereof.

The present invention is thus further embodied by system **60** for mapping irregularities in surface **68** of hard tissue **70** within a target. System **60** includes at least one transducer **62,** a position locator and adjustment mechanism **74** and central processing unit 72.

Transducer **62** is positioned at defined location **64** and is capable of transmitting focused beam **66** of ultrasonic energy towards surface **68** of hard tissue **70** at first angle **76** of incidence. Transducer **62** is further capable of receiving at least a portion **65** of the energy as an echo-reflection from the surface **68** of hard tissue **70.** Transducer **62** is further capable of communication with central processing unit **72** for purposes of transmission and receipt of data. This data may include positional information (e.g. **64** or **76** or 46) as well as commands.

Position locator and adjustment mechanism **74** is operably connectable to, or integrally formed with, transducer(s) **62** and is designed and constructed to be capable of adjusting **29** oblique angle of incidence **76** between focused beam **66** and surface **68** of hard tissue 70 in response to a command from central processing unit **72.**

Position locator and adjustment mechanism **74** is further capable of defining **26** the location of the transducer **62** as a set of position co-ordinates 64 in six degrees of freedom and transmitting the set of co-ordinates **64** to central processing unit **72.** Position locator and adjustment mechanism **74** is further capable of moving **30** transducer **62** to a series of different defined locations **64.**

Central processing unit **72** is designed and configured to be capable of receiving position co-ordinates **64** defining the location of transducer **62** from position locator and adjustment mechanism **74** and to be further capable of calculating **28** an additional set of position co-ordinates **46** for a portion of surface **68** of hard tissue **70** causing echo-reflection **65** and to be further capable of compiling **34** a plurality of the sets of position co-ordinates to generate a map of the surface of the hard tissue.

Central processing unit **72** may be, for example, a computer such as a personal computer (PC) having an operating system such as DOS, Windows^{™}, OS/2^{™} or Linux; Macintosh^{™}, Palm OS^{™}, an EPOC^{™} computer; a computer having JAVA^{™} -OS as the operating system; a graphical workstations such as a computer of Sun Microsystems^{™} or Silicon Graphics^{™}, or another computer having some version of the UNIX operating system such as AIX^{™} or SOLARIS^{™} of Sun Microsystems^{™}; or any other known and available operating system, or a personal digital assistant (PDA), each of which is known to include an inherent or connectable display device **82.**

Optionally, but also preferably, central processing unit **72** is further designed and configured to be capable of transmitting a command to position locator and adjustment mechanism **74** to cause at least one transducer **62** to move **30** to a series of different defined locations **64**. The command may be, for example a command to a mechanical control, a command to switch to a different transducer 62 an array of transducers **62** (see figure4s 3a-c) or a command to an electronic control.

Alternately, but also preferably, position locator and adjustment mechanism **74** is designed and configured to receive input from an operator of system **60.** The input may include, for example, a manual position adjustment of transducer **62** by an operator of system **60.** Alternately or additionally, the input may include at least one instruction transmitted to central processing unit **72** by the operator. Instructions may be transmitted by means of input device **84** which is preferably a part of system **60.** Input device **84** may be any device for entry of data to a computing device such as CPU **72.** Therefore, input device **82** may include, but is not limited to, a keyboard, a computer mouse, a trackpad, a track ball, a stylus, a touchscreen or a microphone.

Thus, method **20** preferably further includes displaying data upon a display device **82** preferably included in system **60.** Display device **82** may include any device which visually presents data to a user. Therefore, display device **82** may be, for example, a cathode ray tube display screen, a liquid crystal display (e.g. active matrix or passive matrix), a plasma screen, a printout or an array of light emitting diodes. The data **44** displayed upon device **82** preferably pertains to echo-reflection **65** and may include one or more set(s) of position co-ordinates **46** corresponding to a portion of the surface **68** of hard tissue **70** causing echo-reflection 65 and/or at least a portion of map **48**. Map **48** is preferably two dimensional.

Thus, the present invention works most effectively when each point or area on surface **68** of hard tissue **70** is examined from a variety of angles of incidence 76 rather than from one or more specific angle of incidence **76.** Further, the greater the number of angles of incidence **76** employed the greater the ability to discern irregularities in surface **68.** Moreover, it is a particular feature of the present invention, that the decision whether or not a certain area on surface **68** of hard tissue **70** area exhibits a pathological discontinuity or inhomogeneity is based on an analysis of echoes of signals transmitted in a wide range of angles of incidence **76** rather than in one or more specific angles of incidence. Thus, the greater the range of angles of incidence **76,** the greater the ability to discern irregularities in surface **68.** As a result, the present invention eliminates the need to determine or estimate the orientation of the hard tissue **70** throughout the scanning procedure, for example, in order to maintain a specific angle of incidence with respect to the hard tissue **70**. This feature is especially advantageous in cases where it is difficult to determine in vivo the orientation of the hard tissue **70** being examined, for example, due to the curved or otherwise untraceable shape of the hard tissue **70** or due to a thick layer of soft tissue interposing between the probe and the hard tissue **70**.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A method of mapping irregularities in a surface of a hard tissue within a target, the method comprising;
(a) transmitting from an ultrasonic transducer (62) at a defined location (64) a focused beam (66) of ultrasonic energy towards the surface (68) of the hard tissue (70) at a first oblique angle of incidence (76);
(b) registering said defined location if and only if an echo-reflection from said surface of the hard tissue is received by said transducer;
(c) defining said location of said transducer in six degrees of freedom;
(d) calculating a set of position co-ordinates (46) for a portion of the surface of the hard tissue causing said echo-reflection;
(e) moving said ultrasonic transducer to a different defined location (64);
(f) repeating a through (e);
(g) repeating steps (a) through (f) with an additional angle at least 20 degrees apart from the previous oblique angle;
(h) determining for each of said set of position co-ordinates for a portion of the surface of the hard tissue causing said echo-reflection at least a portion of a reflection diagram;
(i) further determining for each of said at least a portion of a reflection diagram a degree of normalcy according to a predetermined rule to generate a map of the irregularities in the surface of the tissue;
(j) classifying any of said set of position co-ordinates for a portion of the surface of the hard tissue wherein said at least a portion of a reflection diagram is **characterized by** a low degree of normalcy according to said predetermined rule as belonging to a surface irregularity; and
(k) compiling at least a portion of said sets of position co-ordinates to generate a map (48) of the irregularities in the surface of the hard tissue.

2. The method of claim 1, wherein said repeating includes adjusting said angle of incidence to at least one second angle of incidence.

3. The method of claim 1, further comprising:
(1) displaying upon a display device (82) at least one item selected from the group consisting of
(i) data pertaining to said echo-reflection;
(ii) said set of position co-ordinates for said portion of the surface of the hard tissue causing said echo-reflection; and
(iii) at least a portion of said map.

4. The method of claim 1, further comprising:
(I) controlling, by means of a central processing unit (72), performance of at least a portion of the method.

5. The method of claim 2, further comprising:
(i) controlling, by means of a central processing unit (72), performance of at least a portion of the method.

6. A system for mapping irregularities in a surface of a hard tissue within a target, the system comprising:
(a) at least one ultrasonic transducer (62);
(i) said at least one transducer positioned at a defined location;
(ii) said at least one transducer capable of transmitting a focused beam (66) of ultrasonic energy towards the surface (68) of the hard tissue (70) at a first angle of incidence (76);
(iii) said at least one transducer capable of receiving at least a portion of said energy as an echo-reflection from the surface of the hard tissue; and
(iv) said at least one transducer capable of communication with a central processing unit (72);
(b) a position locator and adjustment mechanism operably connectable to said at least one transducer and designed and constructed;
(i) to be capable of adjusting said angle of incidence between said focused beam and the surface of the hard tissue in response to a command from said central processing unit;
(ii) to be further capable of defining said location of said transducer as a set of position co-ordinates in six degrees of freedom and transmitting said set of coordinates to a central processing unit;
(iii) to be further capable of moving said at least one ultrasonic transducer to a series of different defined location;
(c) said central processing unit designed and configured;
(i) to be further capable of receiving said set of position co-ordinates defining said location of said at least one transducer from said position locator and adjustment mechanism;
(ii) to be further capable of calculating an additional set of position coordinates for a portion of the surface of the hard tissue causing said echo-reflection;
(iii) to be further capable of compiling a plurality of said sets of position coordinates to generate a neap of the surface of the hard tissue.

7. The system of claim 6, further comprising a:
(d) a display device (82) capable of communication with said central processor; said display device designed and constructed to perform at least one function selected from the group consisting of
(i) display data pertaining to said echo-reflection;
(ii) display said additional set of position co-ordinates for a portion of the surface of the hard tissue causing said echo-reflection; and
(iii) display at least a portion of said map.

8. The system of claim 6, wherein said central processing unit is further designed and constructed to be capable of transmitting a command to said position locator and adjustment mechanism to cause said at least one transducer to move to said series of different defined locations;

9. The system of claim 8, wherein said command is selected from the group consisting of a command to a mechanical control, a command to switch to a different transducer of said at least one transducer and a command to an electronic control.

10. The system of claim 6, wherein said position locator and adjustment mechanism is designed and configured to receive input from an operator of the system, said input being selected from the group consisting of a manual position adjustment by an operator of the system and at least one instruction transmitted to said central processing unit (72) by said operator.

## Patentansprüche

1. Verfahren zur Abbildung von Unregelmäßigkeiten in einer Oberfläche eines harten Gewebes innerhalb eines Zielgebiets, wobei zu dem Verfahren die Schritte gehören:
(a) Abstrahlen eines fokussierten Ultraschallenergiestrahls (66) auf die Oberfläche (68) des harten Gewebes (70) unter einem ersten schrägen Einfallswinkel (76) aus einem Ultraschallwandler (62) an einer definierten Position (64);
(b) Aufzeichnen der definierten Position, falls und lediglich falls eine Echoreflexion von der Oberfläche des harten Gewebes von dem Wandler empfangen wird;
(c) Definieren der Position des Wandlers in sechs Freiheitsgraden;
(d) Berechnen eines Satzes von Positionskoordinaten (46) für einen Abschnitt der Oberfläche des harten Gewebes, das die Echoreflexion hervorruft;
(e) Bewegen des Ultraschallwandlers an eine andere definierte Position (64);
(f) Wiederholen der Schritte (a) bis (e);
(g) Wiederholen der Schritte (a) bis (f) mit einem zusätzlichen Winkel, der sich um mindestens 20 Grad von dem vorherigen schrägen Winkel unterscheidet;
(h) Ermitteln wenigstens eines Teils eines Reflexionsdiagramms für jeden der Sätze von Positionskoordinaten für einen Abschnitt der Oberfläche des harten Gewebes, das die Echoreflexion hervorruft;
(i) ferner Ermitteln eines Grades von Normalität für jeden der (mindestens einen) Abschnitte eines Reflexionsdiagramms gemäß einer vorbestimmten Regel, um eine Karte der in der Oberfläche des Gewebes vorhandenen Unregelmäßigkeiten zu erzeugen;
(j) Einstufen jedes der Sätze von Positionskoordinaten für einen Abschnitt der Oberfläche des harten Gewebes, wobei der zumindest eine Abschnitt eines Reflexionsdiagramms **dadurch gekennzeichnet ist, dass** ein geringer Grad von Normalität gemäß der vorbestimmten Regel im Zusammenhang mit einer Oberflächenunregelmäßigkeit steht; und
(k) Kompilieren wenigstens eines Teils der Sätze von Positionskoordinaten, um eine Karte (48) der Unregelmäßigkeiten in der Oberfläche des harten Gewebes zu erzeugen.

2. Verfahren nach Anspruch 1, wobei der Schritt des Wiederholens ein Anpassen des Einfallswinkels an wenigstens einen zweiten Einfallswinkel beinhaltet.

3. Verfahren nach Anspruch 1, zu dem ferner der Schritt gehört:
(1) Anzeigen auf einer Anzeigevorrichtung (82) wenigstens eines Elements, das aus der Gruppe ausgewählt ist, zu der gehören:
(i) Daten, die die Echoreflexion betreffen;
(ii) der Satz von Positionskoordinaten für den Abschnitt der Oberfläche des harten Gewebes, der die Echoreflexion hervorruft; und
(iii) wenigstens ein Abschnitt der Karte.

4. Verfahren nach Anspruch 1, zu dem ferner der Schritt gehört:
(i) Steuern des Ablaufs wenigstens eines Teils des Verfahrens mittels einer Zentraleinheit (72).

5. Verfahren nach Anspruch 2, zu dem ferner der Schritt gehört:
(i) Steuern des Ablaufs wenigstens eines Teils des Verfahrens mittels einer Zentraleinheit (72).

6. System zum Abbilden von Unregelmäßigkeiten in einer Oberfläche eines harten Gewebes innerhalb eines Zielgebiets, wobei zu dem System gehören:
(a) wenigstens einen Ultraschallwandler (62);
(i) wobei der wenigstens eine Wandler an einer definierten Position positioniert ist;
(ii) wobei der wenigstens eine Wandler in der Lage ist, einen fokussierten Ultraschallenergiestrahl (66) unter einem ersten Einfallswinkel (76) gegen die Oberfläche (68) des harten Gewebes (70) abzustrahlen;
(iii) wobei der wenigstens eine Wandler in der Lage ist, wenigstens einen Teil der Energie in Form einer Echoreflexion von der Oberfläche des harten Gewebes zu empfangen; und
(iv) wobei der wenigstens eine Wandler in der Lage ist, Daten mit einer Zentraleinheit (72) auszutauschen;
(b) eine Positionslokalisierungs- und -anpassungseinrichtung, die sich betriebsmäßig an den wenigstens einen Wandler anschließen lässt und dazu entwickelt und konstruiert ist,
(i) in der Lage zu sein, in Reaktion auf einen Befehl von der Zentraleinheit den Einfallswinkel zwischen dem fokussierten Strahl und der Oberfläche des harten Gewebes anzupassen;
(ii) ferner in der Lage zu sein, die Position des Wandlers als einen Satz von Positionskoordinaten in sechs Freiheitsgraden zu definieren und den Satz von Koordinaten an eine Zentraleinheit zu übermitteln;
(iii) ferner in der Lage zu sein, den wenigstens einen Ultraschallwandler zu einer Serie von verschiedenen definierten Positionen zu bewegen;
(c) wobei die Zentraleinheit konstruiert und konfiguriert ist,
(i) ferner in der Lage zu sein, den Satz von Positionskoordinaten, der die Position des wenigstens einen Wandlers definiert, von der Positionslokalisierungs- und - anpassungseinrichtung entgegenzunehmen;
(ii) ferner in der Lage zu sein, einen zusätzlichen Satz von Positionskoordinaten für einen Abschnitt der Oberfläche des harten Gewebes, das die Echoreflexion hervorruft, zu berechnen;
(iii) ferner in der Lage zu sein, mehrere der Sätze von Positionskoordinaten zu kompilieren, um eine Karte der Oberfläche des harten Gewebes zu erzeugen.

7. System nach Anspruch 6, zu dem ferner gehören:
(d) eine Anzeigevorrichtung (82), die in der Lage ist, mit dem Zentralprozessor Daten auszutauschen; wobei die Anzeigevorrichtung dazu entwickelt und konstruiert ist, wenigstens eine Funktion durchzuführen, die aus der Gruppe ausgewählt ist, zu der gehören:
(i) Anzeigen von Daten, die die Echoreflexion betreffen;
(ii) Anzeigen des zusätzlichen Satzes von Positionskoordinaten für einen Abschnitt der Oberfläche des harten Gewebes, das die Echoreflexion hervorruft; und
(iii) Anzeigen wenigstens eines Abschnitts der Karte.

8. System nach Anspruch 6, wobei die Zentraleinheit ferner dazu entwickelt und konstruiert ist, in der Lage zu sein, einen Befehl an die Positionslokalisierungs- und - anpassungseinrichtung zu übermitteln, um zu veranlassen, dass sich der wenigstens eine Wandler zu der Serie unterschiedlicher definierter Positionen bewegt;

9. System nach Anspruch 8, wobei der Befehl aus der Gruppe ausgewählt ist, zu der gehören: ein Befehl an eine mechanische Steuerung, ein Befehl, zu einem anderen Wandler des wenigstens einen Wandlers umzuschalten, und ein Befehl an eine elektronische Steuerung.

10. System nach Anspruch 6, wobei die Positionslokalisierungs- und -anpassungseinrichtung dazu konstruiert und eingerichtet ist, eine Eingabe von einem Anwender des Systems entgegenzunehmen, wobei die Eingabe aus der Gruppe ausgewählt ist, die aus einer durch eine Bedienperson des Systems manuell durchgeführten Positionseinstellung und wenigstens einer durch die Bedienperson an die Zentraleinheit (72) übermittelten Anweisung besteht.

## Revendications

1. Procédé de cartographie des irrégularités d'une surface d'un tissu dur à l'intérieur d'une cible, le procédé comprenant les opérations consistant à :
(a) émettre depuis un transducteur à ultrasons (62) en une position définie (64) un faisceau focalisé (66) d'énergie ultrasonore vers la surface (68) du tissu dur (70) selon un premier angle d'incidence oblique (76) ;
(b) enregistrer ladite position définie si et seulement si une réflexion d'écho de ladite surface du tissu dur est reçue par ledit transducteur ;
(c) définir ladite position dudit transducteur dans six degrés de liberté ;
(d) calculer un ensemble de coordonnées de position (46) pour une partie de la surface du tissu dur provoquant ladite réflexion d'écho ;
(e) déplacer ledit transducteur à ultrasons jusqu'à une position définie différente (64) ;
(f) répéter les étapes (a) à (e);
(g) répéter les étapes (a) à (f) avec un angle supplémentaire d'au moins 20 degrés par rapport au précédent angle oblique ;
(h) déterminer pour chacune des coordonnées de position pour une partie de la surface du tissu dur provoquant ladite réflexion d'écho au moins une partie d'un diagramme de réflexion ;
(i) déterminer en outre pour chacune desdites au moins une partie d'un diagramme de réflexion un degré de normalité selon une règle prédéterminée pour générer une carte des irrégularités de la surface du tissu ;
(j) classer toutes les coordonnées de position dudit ensemble pour une partie de la surface du tissu dur, où ladite au moins une partie d'un diagramme de réflexion est **caractérisée par** un bas degré de normalité selon ladite règle prédéterminée comme appartenant à une irrégularité de surface ; et
(k) compiler au moins une partie desdits ensembles de coordonnées de position pour générer une carte (48) des irrégularités de la surface du tissu dur.

2. Procédé selon la revendication 1, dans lequel ladite répétition comprend le fait de régler ledit angle d'incidence sur au moins un deuxième angle d'incidence.

3. Procédé selon la revendication 1, comprenant en outre les étapes consistant à :
(1) afficher sur un dispositif d'affichage (82) au moins un élément choisi dans le groupe comprenant :
(i) des données appartenant à ladite réflexion d'écho ;
(ii) ledit ensemble de coordonnées de position pour ladite partie de la surface du tissu dur provoquant ladite réflexion d'écho ; et
(iii) au moins une partie de ladite carte.

4. Procédé selon la revendication 1, comprenant en outre l'étape consistant à :
(i) commander, au moyen d'une unité centrale (72), l'exécution d'au moins une partie du procédé.

5. Procédé selon la revendication 2, comprenant en outre l'étape consistant à :
(i) commander, au moyen d'une unité centrale (72), l'exécution d'au moins une partie du procédé.

6. Système permettant de cartographier les irrégularités d'une surface d'un tissu dur à l'intérieur d'une cible, le système comprenant :
(a) au moins un transducteur à ultrasons (62) ;
(i) ledit au moins un transducteur positionné en une position définie ;
(ii) ledit au moins un transducteur capable d'émettre un faisceau focalisé (66) d'énergie ultrasonore vers la surface (68) du tissu dur (70) selon un premier angle d'incidence (76) ;
(iii) ledit au moins un transducteur capable de recevoir au moins une partie de ladite énergie sous forme de réflexion d'écho provenant de la surface du tissu dur ; et
(iv) ledit au moins un transducteur capable de communiquer avec une unité centrale (72) ;
(b) un mécanisme de positionnement et de réglage pouvant être connecté de manière fonctionnelle audit au moins un transducteur et conçu et construit :
(i) pour être capable de régler ledit angle d'incidence entre ledit faisceau focalisé et la surface du tissu dur en réponse à une instruction provenant de ladite unité centrale ;
(ii) pour être en outre capable de définir ladite position dudit transducteur comme un ensemble de coordonnées de position dans six degrés de liberté et de transmettre ledit ensemble de coordonnées à une unité centrale ;
(iii) pour être en outre capable de déplacer ledit au moins un transducteur à ultrasons vers une série de positions définies différentes ;
(c) ladite unité centrale conçue et configurée :
(i) pour être en outre capable de recevoir ledit ensemble de coordonnées de position définissant ladite position dudit au moins un transducteur transmis par ledit mécanisme de positionnement et de réglage ;
(ii) pour être en outre capable de calculer un ensemble de coordonnées de position supplémentaire pour une partie de la surface du tissu dur provoquant ladite réflexion d'écho ;
(iii) pour être en outre capable de compiler une pluralité desdits ensembles de coordonnées de position afin de générer une carte de la surface du tissu dur.

7. Système selon la revendication 6, comprenant en outre :
(d) un dispositif d'affichage (82) capable de communiquer avec ladite unité centrale ; ledit dispositif d'affichage étant conçu et construit pour exécuter au moins une fonction choisie dans le groupe comprenant :
(i) afficher des données appartenant à ladite réflexion d'écho ;
(ii) afficher ledit ensemble supplémentaire de coordonnées de position pour une partie de la surface du tissu dur provoquant ladite réflexion d'écho ; et
(iii) afficher au moins une partie de ladite carte.

8. Système selon la revendication 6, dans lequel ladite unité centrale est en outre conçue et construite pour être capable de transmettre une instruction audit mécanisme de positionnement et de réglage afin de déplacer ledit au moins un transducteur à ultrasons vers ladite série de positions définies différentes.

9. Système selon la revendication 8, dans lequel ladite instruction est choisie dans le groupe comprenant une instruction pour une commande mécanique, une instruction pour passer à un autre transducteur dudit au moins un transducteur et une instruction pour une commande électronique.

10. Système selon la revendication 6, dans lequel ledit mécanisme de positionnement et de réglage est conçu et configuré pour recevoir une entrée d'un opérateur du système, ladite entrée étant choisie dans le groupe comprenant un réglage de position manuel par un opérateur du système et au moins une instruction transmise à ladite unité centrale (72) par ledit opérateur.
